# EUROPEAN PATENT APPLICATION

(11) **EP 4 737 020 A1**
(43) Date of publication of application: **06.05.2026**
(21) Application number: 24383186.4
(22) Date of filing: 30.10.2024
(51) Int. Cl.: B07C 5/342, B07C 5/36, G01N 33/02, G06T 7/00

(54) **COMPUTER-IMPLEMENTED SYSTEM AND METHOD FOR SORTING BANANAS**

(71) Applicant: ESAE Vision System S.L., 38206 La Laguna (Santa Cruz de Tenerife) (ES)
(72) Inventor: Carreira Lorenzo, Adrián, LA LAGUNA (SANTA CRUZ DE TENERIFE) (ES); Lorenzo Dávila, Alejandro, LA LAGUNA (SANTA CRUZ DE TENERIFE) (ES); Fuertes Rodríguez, Miguel Angel, LA LAGUNA (SANTA CRUZ DE TENERIFE) (ES); Moreno Pérez, Javier, LA LAGUNA (SANTA CRUZ DE TENERIFE) (ES)
(74) Representative: Isern Patentes y Marcas S.L.

(57) **Abstract**

The present invention provides a banana sorting system comprising two conveyor belts (1) separated by a gap (2), whereby a banana group (3) is transferred from one belt (1) to another. It includes imaging means (4) that capture the group from different angles, including the gap (2), allowing a series of images to be obtained during the passage thereof. These means are also designed to reconstruct a single image of the hidden areas of the banana group (3) that are not visible when said banana group (3) is resting on the belts (1). The system has a control system (6) that manages both the belts (1) and the imaging means (4), and determines the quality of the banana from the reconstruction of images and the different viewpoints. Based on this evaluation, the system sorts the banana group (3) according to the assigned quality category, allowing an accurate and automated sorting of the bananas.

## Description

### TECHNICAL FIELD OF THE INVENTION

The present invention relates to a system for categorising and sorting bananas. The invention also relates to a computer-implemented method for sorting bananas.

### STATE OF THE ART

One of the most delicate and relevant processes in the value and marketing chain of bananas, particularly Canary Island bananas, is the quality-based sorting of bananas. In this sense, Canary Island bananas are recognised for their quality and their presence on the foreign market depends on this. Quality standards are regulated by the regulations dictated by BOE-A-1987-26231 Order of 23 November 1987 for the Spanish market and by the International Standards for Fruit and Vegetables for bananas (OECD, 2023) for international trade.

According to these regulations, the quality sorting of bananas is mainly based on the presence of damage on the peel, such as rubbing, damage caused by pests, the presence of latex, etc., parameterised per damaged unit area (cm²/banana) and, at present, the detection of the presence of damage is performed by the same methodology used for many years, that is, by means of visual inspection by qualified operators.

This rudimentary sorting-detection technique requires a lot of personnel, making it the highest cost of banana packaging and the second highest cost of the entire value chain of the product, as reflected in the "Estudio de la cadena de valor y formatión de los precios del plátano" (Study of the banana value chain and price formation) (Spanish Ministry of Agriculture, Fish and Food, 2009).

Accordingly, for a packaging company to process 30 million kilos of bananas, around 100 people are required for this process alone, achieving a yield of 133 groups/min as efficiently as possible, where at least 5 packing lines are used to achieve this throughput.

This leads to a bottleneck in the packaging and marketing process, not being able to deliver the goods requested by the market on time or not being able to process the required production volumes due to the intense harvesting season. As it is a completely manual process, subject only to the objective criteria of an operator, a high percentage of errors are made in the sorting that harm the farmer in the remuneration of the fruit and cause great havoc for the packing company due to the return of lots that do not meet the minimum required and/or legal standards.

Therefore, there is a clear need to improve the banana sorting process, making it more efficient while at the same time less dependent on human intervention.

### DESCRIPTION

In order to respond to the detected need, the present invention provides a banana sorting system, hereinafter system, as specified in claim 1. Particular embodiments of the system are set forth in the claims that are dependent on said independent claim 1.

Within the scope of the invention, a bunch of bananas is the name given to the set consisting of hands, which are in turn made up of a plurality of fingers (bananas), said hands hanging from a stem, the bunch being made up of a plurality of hands attached to the stem. However, given that a hand can be whole (with all its fingers) or fragmented, referred to as a cluster, in the banana sorting system, sorting will be carried out by processing one group, which is either a hand or a cluster, interchangeably.

According to the foregoing, in a first inventive aspect, *the banana sorting system comprises:*
- *two conveyor belts separated from one another in a conveying direction with a gap between them, the conveyor belts being configured for conveying at least one group consisting of bananas which is transferred from one belt to the other;*
- *imaging means configured to capture images of the group as it moves along the conveyor belts from different viewpoints including the viewpoint from the gap, such that said imaging means are further configured to capture a series of images of the group* as *it passes through the gap as seen from said gap; and*
- *control means configured to:*
   - *control the conveyor belts and the imaging means;*
   - *obtain an image of a hidden part of the banana group resting against the conveyor belts from reconstructing into a single image the series of images of the banana group as it passes through the gap as seen from said gap;*
   - *determine the category of the banana group according to the quality of the banana group established from the images captured by the imaging means from different angles and the reconstructed single image;*
      *and*
   - *sort the group based on the determined category thereof.*

Firstly, it is important to note that, unlike other fruits, an individual banana and the group have an irregular shape and cavities, which prevents them from turning or rotating in a machine in order to evaluate their condition over their entire outer surface. In addition to this fact, due to the delicate nature of this fruit, unnecessary handling of the banana or group may cause additional involvement thereof, which will therefore affect its categorisation and sorting.

Due to the foregoing, in the proposed system the group is conveyed on the conveyor belts, avoiding the handling thereof, where the imaging means capture images of said group as it moves over the belts. In a conventional belt conveyor system, the portion of the group resting on the belt could not be captured by the imaging means. Advantageously, therefore, in the proposed system, the gap between the belts is arranged to enable the imaging means to capture a series of images of the group as it passes from one belt to another.

Since the gap, clearance or separation between the conveyor belts is such that it allows the group (or banana) to pass from one conveyor belt to the other without falling through it, the portion of the group observable from said gap is limited by the opening thereof. Thus, through the control means, the imaging means, based on the speed of the conveyor belts, capture a series of images of the group as it passes over the gap, where each image of this series will contain a section of this hidden portion or side of the group, such that, in order to obtain a complete image of that hidden portion of the group, the control means, from the series of captured images, reconstructs into a single image the aforementioned series of images.

Advantageously, thanks to the foregoing, images of the group are available from several points, including the one that is hidden because the group is resting on the belt, where this set of images, including the reconstructed single image, are processed by the control means to categorise the group from a determined quality.

With respect to the foregoing, and in a particular embodiment, *in order to determine the quality of the group from the images captured by the imaging means from different angles and the reconstructed single image, the control means are configured to detect the presence of damage per unit area on the peel of the bananas in the banana group and categorise the group according to a quantification of said damage, wherein such damage comprises rubbing and*/*or damage due to pests and*/*or the presence of latex,* among others. Quantifying the damage refers to the degree of involvement of said damage, i.e. the extent thereof.

Imaging means shall be understood to mean any device capable of producing an organised set of data, such as pixels, as a result of an exposure of a sensitive or sensing part to radiation, such as infrared radiation or light. Examples of imaging means are digital cameras, infrared cameras, hyperspectral cameras and the like. In one embodiment, the imaging means comprise one, two or more cameras, pointing at the same or different portions of the surface of the banana group. In a still more preferred embodiment, the imaging means comprise one or more digital cameras pointing at the group from the peak, zenith and nadir viewpoints, where in the latter, one or more cameras are arranged to point from the gap between the conveyor belts, where the digital cameras capture images with a given number of pixels. Even more preferably, cameras can be placed in different positions to take different viewpoints and the lens can be changed to take different angles.

Since each image is made up of a certain number of pixels, the control means are configured to convert such pixels into a unit area (for instance cm²) such that, for instance, the area of the group in the image and, accordingly, its size, as well as the size or extent of the damage and its location, can be determined.

Alternatively, the system comprises a sensor configured to detect both the exact moment the group is captured by the imaging means, i.e. by the zone of the cameras, as well as the length of the group. In a preferred embodiment this sensor is a laser type sensor.

In a particular embodiment, *in order to detect the presence of damage on the peel of the bananas in the banana group and to categorise the group according to a quantification of said damage, the control means are configured to quantify the damage from:*
- *determining the number of fingers in the banana group;*
- *determining which fingers are damaged and the relative positions of the damage on each finger; and*
- *determining the amount of damage per unit area of the peel;*
*wherein the category of the group is determined from comparing the quantified damage with pre-established target values.*

In view of the foregoing, the control means are configured to identify the damage and generate damage-related labels, for instance latex label if latex has been detected, or rubbing label if rubbing is involved, wherein the control means are configured to, for instance, create shapes that circumscribe the detected damage and assign the aforementioned label to it.

Advantageously, from determining the extent per unit area of the group, the control unit can determine the number of fingers (bananas) in the group, as well as the location of the damage and its extent. From the location of the damage and its extent, the quality of the group is determined, whereby making it possible to establish a category of the group which will be used for sorting and subsequent packaging thereof.

In another particular embodiment, *from the images captured by the imaging means from different angles and the reconstructed single image, the control means are further configured to:*
- *identify a crown of the group and determine its condition; and*/*or*
- *determine the level of ripeness based on the colour of the fingers in the group; and*/*or*
- *generate a 3D model of the group.*

In this case, the condition of the crown as well as the level of ripeness of the fingers serve as parameters to determine the quality of the group and to establish its category. In addition, a 3D model of the group is generated in parallel with the set of images, which will help in the process to determine the quality, size, among others, as well as serve as a visual explanation to a user of the system.

In addition to the above parameters, the control means are configured to determine the blossom scar (lower part of each finger or banana), blossom remains, cracked/cut zones, removal of fingers (bananas) in the group, deformations and type of group.

In a particular embodiment, *the control means implement an artificial intelligence trained to determine the category of the banana group according to the quality of the banana group established from the set of images,* wherein the artificial intelligence is configured to detect the presence of damage per unit area on the peel of the fingers or bananas in the group and categorise the group according to a quantification of said damage, wherein such damage comprises rubbing and/or damage caused by pests and/or the presence of latex

With respect to the artificial intelligence, a training dataset of images of banana groups covering the different situations is created to train the artificial intelligence. The dataset must be large, each category and situation must be correctly represented in a subset of the images. Although there is no minimum number needed for training, it has been found that good results are obtained from 1250 images per subset.

As mentioned in previous paragraphs, the control means are prepared to identify damage and generate damage-related labels, such that, with the labelled data, the artificial intelligence model, which in a preferred embodiment is based on a COCO and/or YOLO architecture, is trained to detect patterns that allow categories of what has been labelled to be identified. Therefore, the trained artificial intelligence model is able to recreate these "shapes", "patterns" in an image it has not seen before under a failure threshold accepted by the system, for instance 5-10 %, which is lower than the failure of a sorting operator in the factory.

From the foregoing, the artificial intelligence model outputs a file containing for each image the positions that delimit the calculated shapes. Through this file, the set of images and the distance from the cameras, it is possible to determine when damage is found on a particular finger, the surface with damage, presence of latex, etc.

In another particular embodiment, *the system comprises weighing means configured to weigh the banana, such that sorting of the banana and*/*or subsequent packaging is performed based on the category and weight of the banana.*

Advantageously, each group can be weighed so that the sorting is determined as a combination of the category (based on the damage detected) and the weight of said group. Although weight is a parameter that optionally influences the sorting, it does have a direct implication on the subsequent treatment of the group, in particular its packaging in a box or container. Thus, sorting is achieved by obtaining the weight and categorisation of each group, such that the groups necessary to meet the total weight of the box with the required specifications can be put together. For example: 17 kg box of category I in cluster format. In this way, the system is able to sort the banana groups and to put them together for a specific type of box.

In yet another particular embodiment, *the system comprises a tunnel through which the conveyor belts run at least partially and in which the imaging means are arranged.*

In another embodiment, the invention provides a computer-implemented method for sorting bananas by means of a system for sorting bananas as specified in the second independent claim. Particular embodiments of the method are set forth in the claims that are dependent on said second independent claim.

With respect to the computer-implemented method, said method is performed in a system for sorting bananas, the system comprising:
- *two conveyor belts separated from one another in a conveying direction with a gap between them, the conveyor belts being configured for conveying at least one banana group which is transferred from one belt to the other;*
- *imaging means configured to capture images of the banana group as it moves along the conveyor belts from different viewpoints including the viewpoint from the gap, such that said imaging means are further configured to capture a series of images of the banana group as it passes through the gap as seen from said gap; and*
- *control means configured to control the conveyor belts and the imaging means, and to obtain an image of a hidden part of the banana group resting against the conveyor belts from reconstructing into a single image the series of images of the banana group captured as it passes through the gap as seen from said gap, wherein the control means comprise an artificial intelligence model configured to determine the category of the banana group according to the quality of the banana group established from the images captured by the imaging means from different angles and the reconstructed single image;*
*wherein the method comprises, through the control means, the steps of*
- *feeding the artificial intelligence with the images captured by the imaging means from different angles and the reconstructed single image;*
- *determining by artificial intelligence the category of the banana group according to the quality of the banana group established from the images captured by the imaging means from different angles and the reconstructed single image; and*
- *obtaining a sorting of the banana group by artificial intelligence based on the determined category thereof*

In a preferred embodiment, *in the step of determining the quality of the banana group from the images captured by the imaging means from different angles and the reconstructed single image, the step of detecting, by artificial intelligence, the presence of damage per unit area on the peel of the banana in the banana group and categorising the group according to a quantification of said damage, wherein such damage comprises rubbing and*/*or damage caused by pests and*/*or the presence of latex.*

In another preferred embodiment, *the step of detecting, by artificial intelligence, the presence of damage on the peel of the bananas in the banana group and categorizing the group according to a quantification of said damage comprise the step of, by artificial intelligence, quantifying the damage from:*
- *determining the number of fingers in the banana group;*
- *determining which fingers are damaged and the relative positions of the damage on each finger; and*
- *determining the amount of damage per unit area of the peel;*
*wherein the category of the group is determined from comparing the quantified damage with pre-established target values.*

In yet another embodiment, *in the step from the images captured by the imaging means from different angles and the reconstructed single image, it comprises, by artificial intelligence, the steps of:*
- *identifying a crown in the group; and*/*or*
- *determining the level of ripeness based on the colour of the fingers in the group.*

It will be obvious to the person skilled in the art that, although the invention has been described in reference to a group, hand or cluster of bananas, the described teachings are equally applicable to a single banana or a plurality of bananas being conveyed individually on conveyor belts.

### BRIEF DESCRIPTION OF THE FIGURES

The foregoing and other advantages and features will be more fully understood from the following detailed description of exemplary embodiments with reference to the accompanying drawings, which should be considered by way of illustration and not limitation, wherein:
- Figure 1 is a perspective view inside a banana sorting system showing a banana group before passing through a gap between two conveyor belts.
- Figure 2 is a perspective view of the banana sorting system showing a tunnel covering an area comprising imaging means.

### DETAILED DESCRIPTION OF AN EXEMPLARY EMBODIMENT

In the following detailed description, numerous specific details are set forth as examples to provide a thorough understanding of the relevant teachings. However, it will be apparent to those skilled in the art that the present teachings can be implemented without such details.

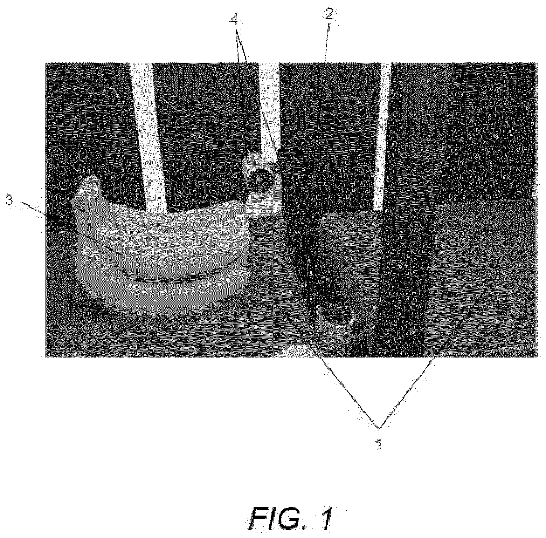

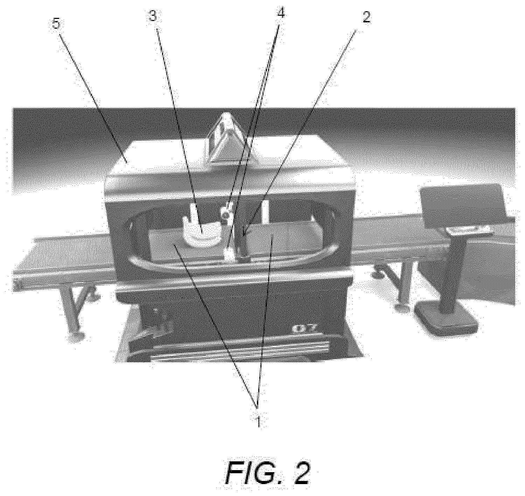

As can be observed in Figures 1 and 2, an embodiment of the present invention can be seen. Two conveyor belts (1) are partially housed inside a tunnel (5), which form a gap (2) between one another configured to pass over the same banana group (3). Also housed inside the tunnel (5) are imaging means (4), which can perceive at least four angles of each banana group passing through same. These angles include one from above, two from the side and one from below through the gap (2) between the conveyor belts (1). The present invention further comprises weighing means (7). In addition, the present invention comprises control means (6) configured to:
- control the conveyor belts (1) and the imaging means (4);
- obtain an image of a part of the banana group (3) resting against the conveyor belts (1) from the reconstruction of a series of images of the banana group (3) captured as it passes over the gap (2), as seen from said gap (2), in a single image;
- determine the category of the banana group (3) according to the quality of the banana group established from the images captured by the imaging means (4) from different angles and the reconstructed single image; and
- sort the banana group (3) based on the determined category thereof.

In order to carry out the present invention and to determine the category of each banana group (3), the following data must be recorded:
- The images of each banana group (3) from the four angles described above;
- The distances of the imaging means (4) to each banana group (3), the distance preferably being in the range of millimetres;
- The speed of movement of the banana groups (3) on the conveyor belts (1);
- The weights of each banana group (3).

The following paragraphs will describe in more detail the different steps and processes necessary to get the banana sorting system up and running, and in particular the definition of the input data.

Taking images by the imaging means (4) is an important aspect of the invention. The objective is to capture images of the banana groups (3) from different angles (above, below, left and right) for subsequent analysis. The imaging means (4), comprising at least four cameras, are strategically positioned on a production line and their positions (above, below, left and right), resolution and focus parameters are configured. The imaging means (4) are synchronised to take images simultaneously and a means of activating the imaging means (4) is implemented to capture the images when a banana group (3) passes through the capture zone. The images taken are stored on a server or in the cloud for subsequent processing. Relevant information such as the timestamp, the banana ID and the angle of capture is attached to each saved image. The saved images are then pre-processed by correcting lighting, cropping and other adjustments. Finally, the control means (6) implement a computer vision algorithm to evaluate the quality of the banana group (3).

As described above, the distance of the imaging means (4) to the banana groups (3) must be determined and adjusted in order to obtain sharp and high quality images. To achieve good positioning of the imaging means (4) and images of the desired quality, firstly, the specifications of the cameras making up the imaging means (4) (for instance focal length, resolution, field of view) are analysed and, secondly, the initial installation data of the imaging means (4) are recorded. The imaging means (4) are then calibrated by measuring and adjusting the physical distance between said means and the conveyor belts (1) in the imaging zone. In order to validate the installation, test images are taken to validate the distance. The final configuration comprises the steps of recording the optimum distances in control means (6) and producing a calibration report containing the details of the calibration.

The speed of movement of the conveyor belts (1) and thus the banana groups (3) must also be calibrated and controlled. The objective of this process is to measure and control the speed of the conveyor belts (1) to ensure synchronisation with the imaging means (4). Firstly, speed sensors are used to measure the speed of the belts and the initial data of the configured speed is recorded. The conveyor belts (1) are then monitored in real time by continuously reading the sensors to obtain speed data and modifying the speed of the conveyor belts (1) if necessary to maintain an effective image capture. Finally, the speed data is stored in a database system and allows automatic adjustment to synchronise the speed of the belts (1) with the imaging means (4).

The weighing of groups (3) is an important aspect of sorting and quality control. Weighing devices (7) located at specific points on the conveyor belt (1) are used and calibrated. Each banana group (3) is weighed as it passes over the weighing means (7) and associated with the ID of the banana group (3). The weights are then recorded in a database, the weight data is used for sorting and quality control, and finally reports on average weight, deviations and anomalies are generated.

The control means (6) are used in order to gather all the data accumulated in the processes described above. Among other functions, the control means (6) act as:
- Central storage, where all recorded data (images, distances, speeds, weights) are stored;
- Control panel, where the operator can view and manage all aspects of the system;
- Controller, where algorithms automatically adjust parameters such as the speed of the belts (1) and camera distances based on real-time data;
- Analyser and report generator, where they use analysis tools that provide new insights into the production process and the quality of banana groups.

Understanding the different important variables in the system, how data is collected from these variables and how they influence the performance of the system, the analysis of the output data will now be explained. Some of the output results or metrics produced are:
- Number of fingers in the group.
- 3D model of the group.
- Damage:
   ∘ Quantity of damage per unit area in cm².
   ∘ Relative positions of the damage with respect to the fingers (to which finger each damage belongs).
   ∘ Type of damage.
   ∘ Cut or cracked fingers
   ∘ Blossom scar
   ∘ Malformations
- Latex:
   ∘ Presence or absence.
   ∘ Relative position of latex (fingers containing latex and surface occupied).
- Identification of the crown.
- Level of ripeness based on colouring.
- Other minor metrics
- Sorting category based on the above data.

Below, a table is included for each metric of interest, detailing the parameters to be evaluated and the results obtained.

**Table 1. Group metrics**

| **Metric** | **Parameters to be evaluated** | **Results obtained** |
|---|---|---|
| **Number of fingers in the group** | - Number of fingers: Automatic counting of fingers in each group using computer vision algorithms. | - Accurate count: Precise number of fingers per group |
| | | - Structure of the group: Validation of the correct configuration of the fingers in each group. |
| | - Distribution: Verification of the arrangement and grouping of the fingers in the group. | |
| **3D model of the group** | - Image capture: Images from multiple angles. | - 3D model: Accurate three-dimensional representation of the banana or group. |
| | - 3D reconstruction: Photogrammetry or laser scanning algorithms to reconstruct the three-dimensional model. | |
| | | - Dimensions: Exact measurements of the volume and shape of the banana. |
| **Amount of damage on the peel per unit area (cm²)** | - Area of damage: Affected surface measured in square centimetres. | - Damaged surface: Accurate measurement of the area affected by damage. |
| | - Edge detection: Algorithms to identify the contours of damaged areas. | |
| **Relative positions of damage with respect to the fingers** | - Location of damage: Precise identification of the position of each damage in relation to the fingers in the group. | - Location of damage: Detailed map of the location of the damage in the group. |
| | - Damage mapping: Assignment of damage to specific fingers. | |
| **Type of damage** | - Sorting of damage: Type of damage (crushed, cut, infection, etc.) sorted by image analysis algorithms. | - Type of damage: Precise identification and sorting of the type of damage. |
| | - Severity of damage: Degree of involvement (mild, moderate, severe). | - Severity of damage: Severity of damage category. |
| **Latex** | - Detection of latex: Visual identification of the presence of latex on the surface. | - Presence of latex: Confirmation of the presence or absence of latex. |
| **Relative position of latex (fingers containing latex)** | - Location of latex: Identification of specific fingers containing latex. | - Distribution of latex: Map of the presence of latex on the fingers in the group. |
| **Identification of the crown** | - Integrity of the crown: Verification of the presence and condition of the crown in the group. | - Condition of the crown: Validation of the integrity and characteristics of the |
| | - Shape and size: Measurement of the shape and dimensions of the crown. | crown. |
| **Level of ripeness based on colouring** | - Colour of the peel: Analysis of colouring of the banana peel using computer vision algorithms. | - Level of ripeness: Sorting of the banana into ripeness categories (green, half-ripe, |
| | - Degree of ripeness: Sorting of the level of ripeness based on the colour scale. | ripe, overripe). |
| **Other minor metrics** | - Curvature: Measurement of the degree of curvature of the banana. | - Curvature: Information on the degree of curvature. |
| | - Length: Measurement of the total length of the banana. | - Length and diameter: Precise dimensions of the |
| | - Diameter: Measurement of the diameter at different points of the banana. | banana. |
| | - Blossom scar. | |
| **Sorting category** | - Sorting algorithm: Use of a model combining all the above metrics to | - Final category: Sorting of bananas into pre- |
| **based on the above data** | determine the category of the banana. | determined categories (for instance Extra, First, Second). |
| | - Weighting of criteria: Assignment of weights to each parameter according to its importance in the final sorting. | |
| | | - Sorting report: Detailed documentation of the sorting process and criteria used. |

On the other hand, it is important to be able to distinguish correctly between natural speckles and defects. To this end, here are four aspects to take into account:
- Colouring: Natural speckles usually have a specific colouring that can be recognised and differentiated from defects. For example, natural speckles may be brown or black but uniform, while defects may be unevenly coloured.
- Shape and Distribution: Natural specks tend to have specific shapes and distributions that are more regular compared to defects, which can be more irregular and inconsistently dispersed.
- Size: Natural speckles are usually smaller and more uniform in size compared to defects, which can vary greatly in size.
- Texture: The texture of natural specks is different from that of defects; specks may be smooth, while defects may be rough or have visible areas of damage.

Each of the above metrics is integrated into control means (6), which provide an exhaustive and detailed analysis of each group, ensuring high precision in the sorting and quality control of the product.

The following paragraphs detail how to train the algorithm. A dataset of images of hands and clusters of bananas should be created to cover the different possibilities. The dataset must be large, each category and situation must be correctly represented in a subset of the images. There is no minimum number required, but preferably there should be at least 1250 images per subset. (An image may belong to more than one subset). The different metrics in each image must then be labelled and identified manually. That is, using auxiliary programmes, creating shapes that enclose the damage, for instance.

With all this labelled data, an artificial intelligence model based on a COCO and/or YOLO architecture must be trained to detect patterns that allow the labelled categories to be identified. The properly trained model is able to create these "shapes" in an image it has not seen before by meeting a predetermined accepted failure threshold. Preferably, the failure threshold should be less than 5-10 %. This is determined by the number of manually sorted images, the quality thereof and the number of training iterations.

The model produces a file containing for each image the positions delimiting the calculated shapes. Through this file, the images and the distance from the cameras, it is possible to determine when damage is found on a particular finger, the surface with damage, the presence of latex, etc.

This part requires not only artificial intelligence algorithms, but also traditional programming and mathematics to correctly calculate relative positions, surfaces and other characteristics extracted from the data. At the same time, a 3D model of the group is generated from the images to aid in the refinement process, as well as to serve as a visual explanation to users.

In addition, it should be noted that a major problem facing the groups is their fragility in handling. Being able to take images from four of the points is complex as it is not possible to rotate the group. Therefore, in the preferred embodiment, a camera is arranged to take 1-3 cm image segments from below through the gap (2) when a small jump is made by the group between the two conveyor belts. These image segments are reconstructed through software as if they were a panoramic image in order to capture the entire group from this viewpoint.

This recomposition algorithm must be correctly optimised, because unlike the other 3 cameras, it must take an N number of images as the group moves along the belt at the conveying speed. These images are then processed and analysed with the trained model. (N is determined by the gap (2) or the distance between the two belts, as well as the dimensions of the group being analysed).

This paragraph describes some of the advantages of the present invention. The in situ identification of the category of a banana group for packaging must be done in a few seconds by a qualified person. This qualified person may suffer from general tiredness or eye strain. In comparison, sorting the images to train a model takes between 2 and 5 minutes per image (4 images per group). This analysis is more thorough and meticulous as there is no assembly line rush to move on to the next group. The results obtained with the whole system, once trained and assembled, allow more reliable results than those produced by a human being to be obtained in less time.

It should be noted that machines offer several advantages such as high accuracy, due to machine vision algorithms that identify details imperceptible to humans; speed, by processing images in milliseconds; consistency, because they do not suffer from eye strain; and processing power, as they can analyse multiple images simultaneously and run complex algorithms efficiently. Operators, moreover, offer flexibility, being able to adjust their criteria in real time and handle complex contexts not predefined in an algorithm. In addition, operators bring intuitive experience, recognising patterns, and can perform manual interventions in unforeseen situations or machine failures.

Furthermore, while in situ identification by operators must be done in seconds due to the speed of the production line, operators may experience fatigue or eye strain, which affects precision and leads to variability in decisions over time. Furthermore, advantageously, sorting images to train a model takes between 2 and 5 minutes per image, allowing for a thorough and accurate evaluation. This ensures more reliable results than humans, and once implemented, the automated system speeds up the sorting process, improving production efficiency.

As indicated above, the analysis and sorting of bananas on a production line offers significant advantages in terms of accuracy, speed and consistency compared to manual inspection by human operators. While humans bring flexibility and intuitive experience, machines overcome the human limitations of errors due to fatigue and variability, ensuring high line performance and reliability in quality control.

## Claims

1. A banana sorting system comprising:
- two conveyor belts separated from one another in a conveying direction with a gap between them, the conveyor belts being configured for conveying at least one banana group which is transferred from one belt to the other;
- imaging means configured to capture images of the group as it moves along the conveyor belts from different viewpoints including the viewpoint from the gap, such that said imaging means are further configured to capture a series of images of the group as it passes through the gap as seen from said gap; and
- control means configured to:
- control the conveyor belts and the imaging means;
- obtain an image of a hidden part of the group resting against the conveyor belts from reconstructing into a single image the series of images of the group as it passes through the gap as seen from said gap;
- determine the category of the group according to the quality of the group established from the images captured by the imaging means from different angles and the reconstructed single image;
and
- sort the group based on the determined category thereof.

2. The banana sorting system according to claim 1, wherein, in order to determine the quality of the group from the images captured by the imaging means from different angles and the reconstructed single image, the control means are configured to detect the presence of damage per unit area on the peel of the bananas in the group and categorise the group according to a quantification of said damage, wherein such damage comprises rubbing and/or damage caused by pests and/or the presence of latex.

3. The banana sorting system according to claim 2, wherein, in order to detect the presence of damage on the peel of the bananas in the group and to categorise the group according to a quantification of said damage, the control means are configured to quantify the damage from:
- determining the number of fingers in the group;
- determining which fingers are damaged and the relative positions of the damage on each finger; and
- determining the amount of damage per unit area of the peel;
wherein the category of the group is determined from comparing the quantified damage with pre-established target values.

4. The banana sorting system according to any of the preceding claims, wherein, from the images captured by the imaging means from different angles and the reconstructed single image, the control means are further configured to:
- identifying a crown in the group; and/or
- determine the level of ripeness based on the colour of the fingers in the group; and/or
- generate a 3D model of the group.

5. The banana sorting system according to any of the preceding claims, wherein the control means implement an artificial intelligence configured to determine the category of the group according to the quality of the group established from the images captured by the imaging means from different angles and the reconstructed single image, detecting the presence of damage on the peel of the fingers in the group, quantifying it and categorising the group according to the quantification of the damage.

6. The banana sorting system according to any of the preceding claims, comprising weighing means configured to weigh the group, such that sorting is performed based on the category and weight of the group.

7. The banana sorting system according to any of the preceding claims, comprising a tunnel through which the conveyor belts run at least partially and in which the imaging means are arranged.

8. A computer-implemented method for sorting bananas by means of a system for sorting bananas, the system comprising:
- two conveyor belts separated from one another in a conveying direction with a gap between them, the conveyor belts being configured for conveying at least one banana group which is transferred from one belt to the other;
- imaging means configured to capture images of the group as it moves along the conveyor belts from different viewpoints including the viewpoint from the gap, such that said imaging means are further configured to capture a series of images of the group as it passes through the gap as seen from said gap; and
- control means configured to control the conveyor belts and the imaging means, and to obtain an image of a hidden part of the group resting against the conveyor belts from reconstructing into a single image the series of images of the group captured as it passes through the gap as seen from said gap, wherein the control means comprise an artificial intelligence model configured to determine the category of the group according to the quality of the group established from the images captured by the imaging means from different angles and the reconstructed single image;
wherein the method comprises, through the control means, the steps of
- feeding the artificial intelligence with the images captured by the imaging means from different angles and the reconstructed single image;
- determining by artificial intelligence the category of the group according to the quality of the group established from the images captured by the imaging means from different angles and the reconstructed single image; and
- obtaining a sorting of the group by artificial intelligence based on the determined category thereof.

9. The method according to claim 8, comprising, in the step of determining the quality of the group from the images captured by the imaging means from different angles and the reconstructed single image, the step of detecting, by artificial intelligence, the presence of damage per unit area on the peel of the banana in the group and categorising the group according to a quantification of said damage, wherein such damage comprises rubbing and/or damage caused by pests and/or the presence of latex.

10. The method according to claim 9, wherein the step of detecting, by artificial intelligence, the presence of damage on the peel of the bananas in the banana group and categorizing the group according to a quantification of said damage comprise the step of, by artificial intelligence, quantifying the damage from:
- determining the number of fingers in the banana group;
- determining which fingers are damaged and the relative positions of the damage on each finger; and
- determining the amount of damage per unit area of the peel;
wherein the category of the group is determined from comparing the quantified damage with pre-established target values.

11. The method according to any of claims 8 to 10, wherein in the step from the images captured by the imaging means from different angles and the reconstructed single image, it comprises, by artificial intelligence, the steps of:
- identifying a crown in the group; and/or
- determining the level of ripeness based on the colour of the fingers in the group.
